# EUROPEAN PATENT SPECIFICATION

(11) **EP 0 723 465 B1**
(45) Date of publication and mention of the grant of the patent: **09.02.2000**
(21) Application number: 94926259.6
(22) Date of filing: 16.09.1994
(51) Int. Cl.: A61M 5/50, A61M 37/00, A61M 31/00, A61M 36/12

(54) **IMPLANT INJECTION DEVICE**
INJEKTIONSVORRICHTUNG FÜR IMPLANTATE
DISPOSITIF D'INJECTION D'IMPLANTS

(30) Priority: 13.10.1993 FI 934513
(43) Date of publication of application: 31.07.1996
(73) Proprietor: LEIRAS OY, 20101 Turku (FI)
(72) Inventor: ALLONEN, Hannu, FIN-21610 Kirjala (FI); LANKINEN, Pekka, FIN-20610 Turku (FI); LEHTINEN, Matti, FIN-20760 Piispanristi (FI)
(74) Representative: Öhman, Ann-Marie
(86) International application number: PCT/FI94/00407
(87) International publication number: WO 95/10314

(56) References cited:
- EP-A- 0 304 107
- EP-A- 0 304 700
- WO-A-88/06905
- GB-A- 2 199 247
- US-A- 3 934 586
- US-A- 4 367 738
- US-A- 4 451 254
- US-A- 4 601 699
- US-A- 4 932 941
- US-A- 5 021 047

## Description

This invention relates to an injection device for once-only use for injecting implants.

Devices for the injection of implants have been described earlier in the patent literature. As examples of patent publications disclosing various injection devices for implants EP 304700, EP 304107, WO 8806905, US 4451254 and GB 2199247 can be mentioned. EP 304700 discloses a device whose sterility was improved by preventing the plunger from coming off the housing accidentally. The housing is made of plastic while the cannula and the plunger are made of metal. EP 304107 discloses a device intended for once-only use for injecting implants in which device the housing is made of plastic while the cannula is made of metal. The patent relates particularly to an element associated with the plunger, the function of which element is to prevent the implant from being pushed forwards too early, i.e. during the puncture of the skin. WO 8806905 relates to a complicated device intended for repeated use for subcutaneous implantation in a desired manner of a plurality of successive implants positioned in said device. The invention relates particularly to the equipment for the administration of the implants. US 4451254 discloses also an implanter for the application of several implants, the said implanter being intended for repeated use, wherein the implants are fed from a cartridge mounted on the side of the implanter. The publication GB 2199247 describes an equipment for the implantation of hormone implants wherein the device, which is intended for once-only use, is entirely made of plastic. An incision is first made into the skin with a scalpel after which a trocar attached to the cannula is forced into a desired depth beneath the skin. The trocar, the other end of which is blunt, is withdrawn and reversed and again inserted in the cannula with the blunt end first so that it comes into contact with the implant and actuates as a plunger.

The implant injection devices described above exhibit several disadvantages and faults.

Devices intended for once-only use involve the risk that misusers of drugs and related substances may get hold of them. The device intended for once-only use described in the European patent publication EP 304107 would be quite easy to use after the removal of its original contents of implants. The plunger can be freely withdrawn from the container of medicinals after which the container can be easily loaded with new substances. The injection device for implants described in the patent publication EP 304700 contains a flexible ring or collar fitted onto the inner surface of the drug container, said ring interacting with an annular groove in the plunger. These elements retard the reciprocating movement of the plunger in the drug container so that the plunger will not come off the drug container accidentally. These members also prevent the plunger from being accidentally displaced forwards before the implants are to be administered. However, if greater pushing or pulling forces are applied to the plunger, the plunger can be displaced in both directions and nothing prevents the plunger from being completely pulled out. No means has been provided to prevent the reuse of the device intended for once-only use described in GB 2199247.

The sterilization of the devices for once-only use is preferably accomplished by gas, e.g. ethylene oxide gas, at the stage when all the components including the implants are disposed in the implant container. Sterilization by gamma radiation represents in principle another alternative for sterilization. It suffers, however, from the disadvantage that it is not tolerated by all pharmaceutical substances. Gas sterilization has much wider applications. The disposable device according to patent EP 304107 cannot be sterilized by gas after assembly of the parts because there are no passages through which the gas could penetrate into the interior of the device.

The manufacturing costs of devices for once-only use must be low. It would be desirable to manufacture all components of the device of plastic. The cannula creates a problem as it must be equipped with a sharp edge for the incision of the skin. In all the constructions described in prior art with the exception of the decive disclosed in GB 2199247 the cannula is made of metal. The device for once-only use according to EP 304107 is of plastic except for the cannula which had to be made of metal.

The objective of this invention is to overcome the problems described above and to provide a novel disposable injection device for implants which does not suffer from the drawbacks disclosed above.

Thus, the object of the present invention is an implant injection device for once-only use, the said device comprising an elongated implant housing or container, a cannula and a plunger or obturator, which is longitudinally displaceable in the implant housing. The device is characterized in that the surface of the plunger and the inner wall of the implant housing are equipped with interacting elements which allow the plunger to be displaced inwards into the implant housing while preventing the removal of the plunger from the implant housing.

The invention provides a safe disposable injection device for implants which after use, if in the hands of unauthorized persons, would no longer be useful for the administration of any substances because the plunger cannot be removed from the implant housing without destroying it.

According to another preferred embodiment of the invention the implant housing, the cannula and the plunger are made of plastic. To protect the cannula before use it is preferably equipped with a protecting cap which can also be made of plastic.

According to a third preferred embodiment the wall of the implant container and the cannula as well as the protecting cap are perforated, which allows the sterilization gases to penetrate. This construction allows gas sterilization of the complete device preloaded with implants.

According to a fourth preferred embodiment an incision edge or blade is attached to the body of the device, e.g. to the protecting cap. The incision blade is used to pierce the skin before the plastic cannula is forced beneath the skin. In order to protect the incision blade before and after use, a projecting member has been construed in close proximity to the incision blade.

The invention will be explained in more detail by reference to the attached drawings wherein
- Figure 1: illustrates a side view of the of the implant housing and the attached cannula
- Figure 2: shows a longitudinal section of the device of Figure 1 loaded with implants
- Figure 3: shows a longitudinal section of the plunger of the device
- Figure 4: shows a side view of the implant housing and the cannula, made in one piece according to another embodiment
- Figure 5: shows a longitudinal section of the device of Figure 4
- Figure 6: shows a longitudinal section of the protecting cap for the cannula, the extremity of said protecting cap being equipped with an incision blade and a projecting member for the protection of the incision blade
- Figure 7: shows a side view of the device of Figure 6 seen from the side of the blade
- Figure 8: shows the device of Figure 7 according to another embodiment
- Figure 9: shows a cross-section of the element surrounding the plunger according to one embodiment
- Figure 10: shows a cross section of the element surrounding the plunger according to another embodiment

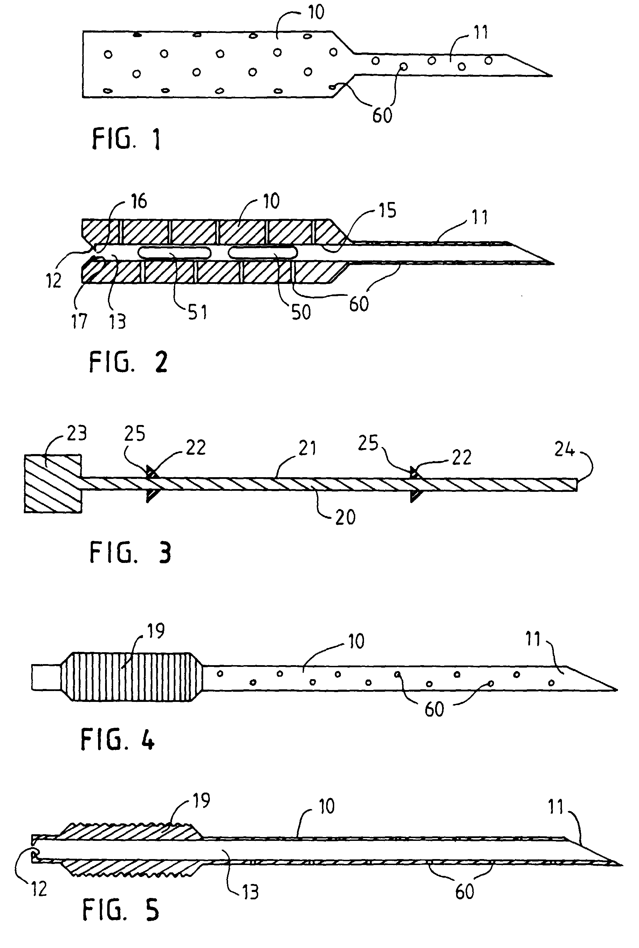

Figure 1 shows a side view of the implant housing 10 and the attached cannula 11. The implant housing as well as the cannula are provided with holes 60 to allow gas sterilization of the device. The implant housing and the cannula can be manufactured separately and joined to each other later. Alternatively these components can be made in one piece. Figures 4 and 5 show a construction where the implant housing and the cannula are integral in one piece so that one end of the housing forms the cannula. Number 19 refers to the handle.

Figure 2 shows a longitudinal section of the implant housing and the attached cannula wherein the implant housing is loaded with two implants 50 and 51. One end 24 of the plunger 20 in Figure 3 is pushed into the bore 13 of the implant housing and the other end of the plunger is shaped as a pushknob 23. The surface 21 of the plunger has been equipped with elements 22 which surround the plunger. These elements 22 may be attached to the plunger later at a separate stage or, alternatively, the plunger and the elements 22 may be manufactured in one piece. The purpose of the elements 22 is to prevent the plunger from being removed from the implant housing. Onto the extremity of the implant housing distal to the cannula a rim or edge 12 has been shaped at position 16, at which point the cross-sectional area of the bore 13 in the implant housing is reduced. The elements 22 have been shaped to bend towards the pushknob when the plunger is pushed inwards into the the implant housing and are therefore able to pass through the reduced area 16. On the other hand, if one tries to withdraw the plunger out of the implant housing the surface 25 of the element 22 comes into contact with the surface 17 of the edge in the reduced area. The movement of the plunger is stopped because the elements 22 cannot bend enough towards the cannula to pass through the reduced area 16. Only one element 22 may be provided, preferably two as shown in Figure 3, or even more. When the element 22 in proximity to the cannula has been pushed into the implant housing it prevents the plunger from coming off during the actuation of the device. After the last element 22, i.e. the one in proximity to the pushknob 23 has been pushed into the implant housing it prevents the reuse of the device for the injection of other substances because the plunger cannot be removed from the implant housing. Preferably the cross-section of the element 22 is shaped approximately as a half-dovetail which broadens towards the pushknob 23 of the plunger.

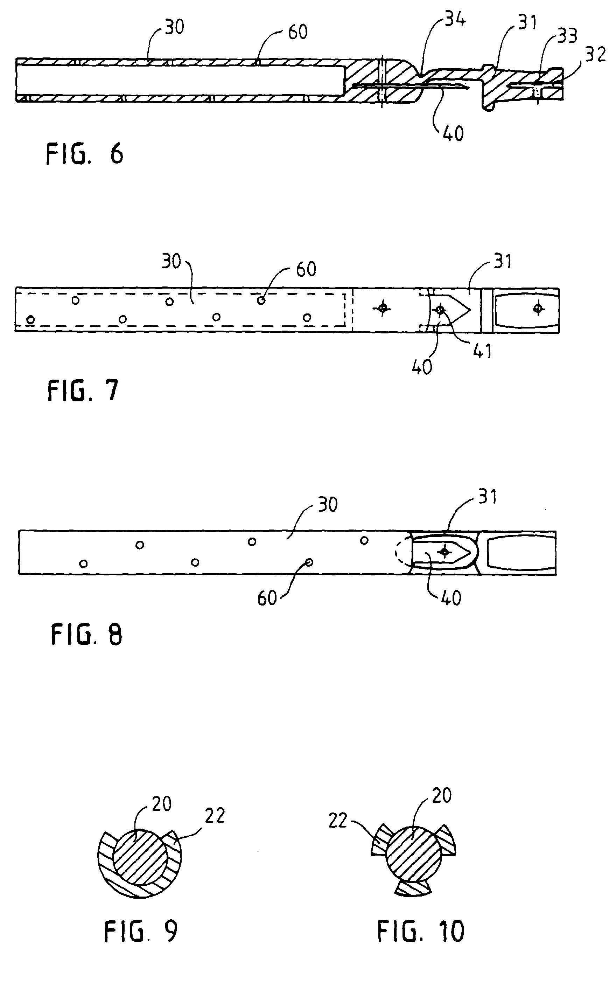

The element 22 can be a continuous annular piece surrounding the outer surface 21 of the plunger. Alternatively it can consist of one or several parts attached to the surface 21 of the plunger to form a surrounding open or discontinuous ring or collar as shown in Figures 9 and 10.

The material of the element 22 must be flexible enough to allow the plunger to be pushed into the implant housing. On the other hand the material must be stiff enough to prevent the plunger from being removed therefrom.

Figure 6 shows the protecting cap 30 for the cannula which cap is likewise provided with holes 60 in order to allow sterilization by gas. Because the cannula 11 is made of plastic it is not by itself sharp enough to effect an incision in the skin and therefore a separate device is needed. For this purpose a separate incision edge or blade 40 has been attached to the extremity of the protecting cap 30. For the protection of the incision blade a projecting member 31 has been provided in close proximity to the incision blade. The projecting member partially surrounds the tip of the blade 40. The projecting member is preferably integral with the protecting cap 30. When the device is taken into use the projecting member 31 is bent upwards, i.e. in the direction away from the blade 40, after which it can be broken along the tearstrip at position 34. After an incision has been made in the skin with the blade 40, the blade 40 can be pushed into the groove 32 of the released protecting member 31. The groove 32 is equipped with an engaging element 33 (for example a knob) which engages with a corresponding element 41 (for example a hole) provided in the blade 40. In this way the blade 40 is protected after use and it will not be dangerous when being destroyed.

In the construction shown by Figures 6 and 7 the projecting member 31 which protects the incision blade forms an integral piece with the protecting cap on one side of the blade (in Figure 6, above the blade). Figure 8 illustrates another construction wherein the projecting member 31 forms a semi-circular element around the incision blade 40.

The incision blade 40 and the projecting member 31 protecting it can also be attached to some other part of the body of the device, e.g. the implant housing or the pushknob of the plunger, because the blade is well protected after use in the groove of the projecting member. The most suitable position for the blade is the protecting cap of the cannula because this component is not used during the injection.

According to the construction described above the element 22 which is flexible to some extent has been attached to the plunger. Alternatively the implant housing can also be equipped with the flexible elements while the plunger can be equipped with a rigid element.

The implant housing and the plunger can also be equipped with elements for the counting of implants administered, for example in the following manner: The plunger can be equipped with two or more ribs or pivots at a distance from each other corresponding to the length of one implant. The pivots project radially from the surface of the plunger in different directions. The edge or rim 12 of the implant housing is equipped with a groove (recess, indentation) running in the axial direction of the implant housing. The plunger can only be pushed into the implant housing by rotating the plunger so that the first rib engages in the groove. When the plunger has been pushed a certain distance into the implant housing, for example a distance corresponding to the length of one implant, the plunger will be stopped because a second pivot projecting in a different direction will abut against the edge 12. Only by rotating the plunger so that the second pivot engages in the groove in the edge 12 the plunger can be pushed further into the implant housing. In this way the unintentional administration of too many implants can be prevented.

Suitable plastic materials for the device according to the invention are any non-toxic, sterilizable plastic materials with a sufficiently stiff structure.

The device according to the invention is intended particularly for the injection of hormone-containing implants to be used for prolonged hormonal treatment or as contraceptives. The final product is preferably supplied loaded with one or several implants.

Those versed in the art will appreciate that many different variations and adaptations of the present invention fall within the scope of the claims to be presented below.

## Claims

1. An implant injection device for once-only use comprising an elongated implant container (10), a cannula (11) and a plunger (20) which is displaced longitudinally in the implant housing, wherein the implant container (10), the plunger (20) and the cannula (11) are all made of plastic, **characterized** in that the surface (21) of the plunger and the inner wall (15) of the implant housing are equipped with interacting members (22, 12) which allow the plunger to be pushed inwards into the implant housing while preventing the removal of the plunger from the implant housing, and in that an incision blade or edge (40) is attached to the body (30, 10 or 20) of said device.

2. The device according to claim 1 **characterized** in that the body of the device further comprises a protecting cap (30) for the protection of the cannula, and the incision blade or edge (40) is attached to said protecting cap (30).

3. The device according to claim 1 or 2 **characterized** in that the surface (21) of the plunger is equipped with a member (22) which allows the displacement of the plunger inwards into the implant housing and that the rim or edge 12 of the implant housing distal to the cannula is shaped so that the inner cross-sectional area of the implant housing is reduced at point 16, and the said edge acts as a stop for the element (22) on the surface of the plunger when the plunger is displaced outwards.

4. The device according to claim 3 **characterized** in that the longitudinal section of the element (22) attached to the surface (21) of the plunger is shaped approximately as a half-dovetail which broadens towards the pushknob (23) of the plunger.

5. The device according to claim 3 or 4 **characterized** in that the element (22) is a continuous annular piece surrounding the surface (21) of the plunger.

6. The device according to claim 3 or 4 **characterized** in that the element (22) consists of one or several parts attached to the surface (21) of the plunger to form a surrounding open or discontinuous ring.

7. The device according to claim 3 or 4 **characterized** in that several elements (22) are attached to the surface (21) of the plunger along its length.

8. The device according to claim 1 **characterized** in that the surfaces of the implant housing and the cannula are provided with holes (60) through which sterilization gases can diffuse.

9. The device according to claim 2 **characterized** in that the protecting cap (30) is perforated to allow penetration of sterilization gases.

10. The device according to claim 1 or 2 **characterized** in that a projecting member (31), extending over and beyond the incision blade (40), has been disposed in close proximity to the said blade in order to protect it.

11. The device according to claim 10 **characterized** in that the extremity of the projecting member (31) is at least partially bent around the tip of the incision blade (40).

12. The device according to claim 11 **characterized** in that the projecting member (31), which forms an integral piece with the body (30, 10 or 20) of the device, is disposed to be easily released from said body for example by tearing along the tearstrip (34).

13. The device according to claim 12 characterized by a recess or groove (32) disposed in the projecting member (31), said groove corresponding to the shape of the incision blade and by a member (33) disposed on the inner surface of said groove, said member engaging with a corresponding element (41) disposed on the incision blade whereupon the incision blade is protected, after use, in the groove of the projecting member.

14. The device according to claim 10 **characterized** in that the incision blade (40) and the projecting member (31) are disposed at the extremity of the protecting cap (30).

15. A combination comprising a device according to any one of the claims 1 - 14 and at least one injectable implant (50 or 51) situated therein.

16. The combination according to claim 15 **characterized** in that the implant (50 or 51) is a hormone-containing implant intended for prolonged hormonal treatment or as a contraceptive.

17. A packaged combination comprising a combination according to claim 15 or 16 wherein said combination is sterilized, and a packaging material in which said sterilized combination is hermetically sealed.

## Patentansprüche

1. Implantatinjektionseinrichtung für nur einmaligen Gebrauch, umfassend einen langgestreckten Implantatbehälter (10), eine Kanüle (11) und einen Kolben (20), welcher der Länge nach in dem Implantatgehäuse verlagerbar ist, worin der Implantatbehälter (10), der Kolben (20) und die Kanüle (11) alle aus Plastik bzw. Kunststoff hergestellt sind, dadurch **gekennzeichnet**, daß die Oberfläche (21) des Kolbens und die innere Wand (15) des Implantatgehäuses mit miteinander in Wechselwirkung tretenden Teilen (22, 12) ausgerüstet sind, welche es erlauben, den Kolben einwärts in das Implantatgehäuse zu schieben, während sie das Entfernen des Kolbens aus dem Implantatgehäuse verhindern, und daß eine Einschnittklinge oder -kante (40) an dem Körper (30, 10 oder 20) der Einrichtung angebracht ist.

2. Einrichtung gemäß Anspruch 1, dadurch **gekennzeichnet**, daß der Körper der Einrichtung weiter eine Schutzkappe (30) für den Schutz der Kanüle umfaßt, und die Einschnittklinge oder -kante (40) an der Schutzkappe (30) angebracht ist.

3. Einrichtung gemäß Anspruch 1 oder 2, dadurch **gekennzeichnet,** daß die Oberfläche (21) des Kolbens mit einem Teil (22) ausgerüstet ist, welches die Verlagerung des Kolbens einwärts in das Implantatgehäuse erlaubt, und daß der Rand oder die Kante (12) des Implantatgehäuses, der bzw. die distal zu der Kanüle ist, so geformt ist, daß der innere Querschnittsbereich des Implantatgehäuses an der Stelle (16) reduziert ist, und die genannte Kante bzw. der genannte Rand als ein Anschlag für das Element (22) auf der Oberfläche des Kolbens wirkt, wenn der Kolben nach auswärts verlagert wird.

4. Einrichtung gemäß Anspruch 3, dadurch **gekennzeichnet**, daß der Längsabschnitt des Elements (22), das an der Oberfläche (21) des Kolbens angebracht ist, angenähert als ein Halbschwalbenschwanz geformt ist, welcher sich nach dem Druckknopf (23) des Kolbens zu verbreitert.

5. Einrichtung gemäß Anspruch 3 oder 4, dadurch **gekennzeichnet,** daß das Element (22) ein kontinuierliches ringförmiges Teil ist, welches die Oberfläche (21) des Kolbens umgibt.

6. Einrichtung gemäß Anspruch 3 oder 4, dadurch **gekennzeichnet**, daß das Element (22) aus einem oder mehreren Teilen besteht, die an der Oberfläche (21) des Kolbens angebracht sind, so daß sie einen umgebenden offenen oder diskontinuierlichen Ring bilden.

7. Einrichtung gemäß Anspruch 3 oder 4, dadurch **gekennzeichnet**, daß mehrere Elemente (22) an der Oberfläche (21) des Kolbens entlang dessen Länge angebracht sind.

8. Einrichtung gemäß Anspruch 1, dadurch **gekennzeichnet**, daß die Oberflächen des Implantatgehäuses und der Kanüle mit Löchern (60) versehen sind, durch welche Sterilisationsgase hindurchdringen bzw. diffundieren können.

9. Einrichtung gemäß Anspruch 2, dadurch **gekennzeichnet**, daß die Schutzkappe 30 zum Ermöglichen eines Ein- bzw. Durchdringens von Sterilisationsgasen perforiert ist.

10. Einrichtung gemäß Anspruch 1 oder 2, dadurch **gekennzeichnet**, daß ein vorstehendes Teil (31), das sich über die und jenseits der Einschnittklinge (40) erstreckt, in enger Nähe zu der Klinge angeordnet worden ist, um sie zu schützen.

11. Einrichtung gemäß Anspruch 10, dadurch **gekennzeichnet**, daß das Ende des vorstehenden Teils (31) wenigstens teilweise um die Spitze der Einschnittklinge (40) herumgebogen ist.

12. Einrichtung gemäß Anspruch 11, dadurch **gekennzeichnet**, daß das vorstehende Teil (31), welches ein integrales Teil mit dem Körper (30, 10 oder 20) der Einrichtung bildet, so angeordnet ist, daß es leicht von dem Körper gelöst werden kann, z.B. durch Ziehen längs des Reißstreifens (34).

13. Einrichtung gemäß Anspruch 12, **gekennzeichnet** durch eine Vertiefung oder Nut (32), die in dem vorstehenden Teil (31) angeordnet ist, wobei die Vertiefung bzw. Nut der Form der Einschnittklinge entspricht, und durch ein Teil (33), das auf der inneren Oberfläche der Vertiefung bzw. Nut angeordnet ist, wobei das genannte Teil mit einem entsprechenden Element (41), welches auf der Einschnittklinge angeordnet ist, in Eingriff tritt, woraufhin die Einschnittklinge nach dem Gebrauch in der Vertiefung bzw. Nut des vorstehenden Teils geschützt ist.

14. Einrichtung gemäß Anspruch 10, dadurch **gekennzeichnet**, daß die Einschnittklinge (40) und das vorstehende Teil (31) an dem Ende der Schutzkappe (30) angeordnet sind.

15. Kombination, umfassend eine Einrichtung gemäß irgendeinem der Ansprüche 1 bis 14 und wenigstens ein injizierbares Implantat (50 oder 51), das sich darin befindet.

16. Kombination gemäß Anspruch 15, dadurch **gekennzeichnet**, daß das Implantat (50 oder 51) ein Hormon enthaltendes Implantat ist, welches zur längeren Hormonbehandlung oder als Contraceptivum dient.

17. Verpackte Kombination, umfassend eine Kombiantion gemäß Anspruch 15 oder 16, worin die genannte Kombination sterilisiert ist, und ein Verpackungsmaterial, in welchem die sterilisierte Kombination hermetisch verschlossen bzw. abgedichtet ist.

## Revendications

1. Dispositif d'injection d'implant à usage unique comprenant un réservoir d'implant allongé (10), une canule (11) et un piston (20) qui peut se déplacer longitudinalement dans le logement d'implant, dans lequel le réservoir d'implant (10), le piston (20) et la canule (11) sont tous faits de matière plastique, caractérisé en ce que la surface (21) du piston et la paroi interne (15) du logement d'implant sont équipés d'éléments interactifs (22, 12) permettant au piston d'être poussé vers l'intérieur dans le logement d'implant tout en empêchant le retrait du piston d'avec le logement d'implant, et en ce qu'une lame ou un tranchant d'incision (40) est fixé au corps (30, 10 ou 20) dudit dispositif.

2. Dispositif selon la revendication 1 caractérisé en ce que le corps du dispositif comprend de plus un capuchon de protection (30) pour la protection de la canule, et la lame ou tranchant d'incision (40) est fixé audit capuchon de protection (30).

3. Dispositif selon la revendication 1 ou 2 caractérisé en ce que la surface (21) du piston est équipée d'un élément (22) permettant le déplacement du piston vers l'intérieur dans le logement d'implant, et en ce que le bord ou l'arête 12 du logement d'implant distal à la canule est façonnée de sorte que la surface en coupe droite du logement d'implant est réduite au niveau du point 16, et ladite arête agit comme point d'arrêt pour l'élément (22) sur la surface du piston lorsque le piston est déplacé vers l'extérieur.

4. Dispositif selon la revendication 3 caractérisé en ce que la section longitudinale de l'élément (22) fixée à la surface (21) du piston est façonnée approximativement comme une demie queue-d'aronde qui s'élargie vers le bouton poussoir (23) du piston.

5. Dispositif selon la revendication 3 ou 4 caractérisé en ce que l'élément (22) est une pièce en forme d'anneau continu entourant la surface (21) du piston.

6. Dispositif selon la revendication 3 ou 4 caractérisé en ce que l'élément (22) est composé d'une ou de plusieurs pièces fixées à la surface (21) du piston pour former un anneau entourant ouvert ou discontinu.

7. Dispositif selon la revendication 3 ou 4 caractérisé en ce que plusieurs éléments (22) sont fixés à la surface (21) du piston sur sa longueur.

8. Dispositif selon la revendication 1 caractérisé en ce que les surfaces du logement d'implant et la canule sont fournies avec des trous (60) au travers desquels des gaz de stérilisation peuvent diffuser.

9. Dispositif selon la revendication 2 caractérisé en ce que le capuchon de protection (30) est perforé pour permettre la pénétration des gaz de stérilisation.

10. Dispositif selon la revendication 1 ou 2 caractérisé en ce que l'on a disposé un élément en saillie (31), s'étendant sur et au-delà de la lame d'incision (40), à proximité de ladite lame afin de la protéger.

11. Dispositif selon la revendication 10 caractérisé en ce que l'extrémité de l'élément en saillie (31) est pliée au moins partiellement autour de la pointe de la lame d'incision (40).

12. Dispositif selon la revendication 11 caractérisé en ce que l'élément en saillie (31), qui forme une seule pièce intégrale avec le corps (30, 10 ou 20) du dispositif, est disposé de manière à être facilement retiré du dit corps par exemple en déchirant le long de la bandelette déchirable (34).

13. Dispositif selon la revendication 12 caractérisé par un évidement ou une rainure (32) disposée dans l'élément en saillie (31), ladite rainure correspondant à la forme de la lame d'incision, et par un élément (33) disposé sur la surface intérieure de ladite rainure, ledit élément venant en prise avec un élément correspondant (41) disposé sur la lame d'incision, ainsi la lame d'incision est protégée, après usage, dans la rainure de l'élément en saillie.

14. Dispositif selon la revendication 10 caractérisé en ce que la lame d'incision (40) et l'élément en saillie (31) sont disposés à l'extrémité du capuchon de protection (30).

15. Combinaison comprenant un dispositif selon l'une quelconque des revendications 1 à 14 et au moins un implant injectable (50 ou 51) situé à l'intérieur.

16. Combinaison selon la revendication 15 caractérisé en ce que l'implant (50 ou 51) est un implant contenant une hormone destinée à un traitement hormonal prolongé ou en tant que contraceptif.

17. Combinaison emballée comprenant une combinaison selon la revendication 15 ou 16 dans laquelle ladite combinaison est stérilisée, et une matière d'emballage dans laquelle ladite combinaison stérilisée est hermétiquement scellée.
